**Europäisches Patentamt**

**European Patent Office**

**Office européen des brevets**

(19)

(11) Veröffentlichungsnummer : **0 352 667 B1**

(12) **EUROPÄISCHE PATENTSCHRIFT**

(45) Veröffentlichungstag der Patentschrift :
**13.05.92 Patentblatt 92/20**

(51) Int. Cl.[5] : **C07C 69/30,** C07C 69/58,
C07C 67/48

(21) Anmeldenummer : **89113478.5**

(22) Anmeldetag : **21.07.89**

(54) **Verfahren zur Herstellung von reinen Monoglyceriden und reinen Diglyceriden aus einem Gemisch, das Mono-, Di- undTriglyceride enthält.**

(30) Priorität : **25.07.88 DE 3825248**

(43) Veröffentlichungstag der Anmeldung :
**31.01.90 Patentblatt 90/05**

(45) Bekanntmachung des Hinweises auf die
Patenterteilung :
**13.05.92 Patentblatt 92/20**

(84) Benannte Vertragsstaaten :
**AT BE CH DE ES FR GB GR IT LI LU NL SE**

(56) Entgegenhaltungen :
**US-A- 4 281 171**
**CHEMICAL ABSTRACTS, Band 97, Nr. 19, 8.**
**November 1982, Columbus, Ohio, USA; ANON,**
**"Extraction of hops with carbon dioxide ina**
**hypercritical state" Seite 550, Spalte 2, Zu-**
**sammenfassung Nr. 161 016c**
**SOVIET INVENTIONS ILLUSTRATED, Sektion**
**Ch, Woche B25, 1. August 1979; DERWENT**
**PUBLICATIONS LTD., London, X25; & SU**
**620474**

(73) Patentinhaber : **Peter, Siegfried, Prof. Dr.**
**Lindenweg 3**
**W-8525 Uttenreuth-Weiher (DE)**

(72) Erfinder : **Peter, Siegfried, Prof. Dr.**
**Lindenweg 3**
**W-8525 Uttenreuth-Weiher (DE)**
Erfinder : **Czech, Bernd, Dipl.-Ing.**
**Eskilstunastrasse 34**
**W-8520 Erlangen (DE)**
Erfinder : **Ender, Ulrich, Dipl.-Ing.**
**St. Johann 6**
**W-8520 Erlangen (DE)**
Erfinder : **Weidner, Dr. Dipl.-Ing.**
**Am Dorfweiher 9**
**W-8520 Erlangen (DE)**

(74) Vertreter : **Weinhold, Peter, Dr. et al**
**Patentanwälte Dipl.-Ing. G. Dannenberg Dr. P.**
**Weinhold Dr. D. Gudel Dipl.-Ing. S. Schubert**
**Dr. P. Barz Siegfriedstrasse 8**
**W-8000 München 40 (DE)**

EP 0 352 667 B1

**Beschreibung**

Monoglyceride sind partielle Ester des Glycerins mit höhermolekularen Fettsäuren. Handelsübliche Monoglyceride bestehen aus Mischungen von Mono- und Di-estern mit geringen Beimischungen von Tri-estern und sind durch Umesterung von Triglyceriden mit Glycerin oder durch Umsetzung von Glycerin mit Fettsäuren erhältlich.

Monoglyceride haben emulgierende, stabilisierende, plastifizierende und verdickende Eigenschaften. Da die Mono- und Di-ester des Glycerins eßbar sind, werden sie in verschiedenen Zweigen der Nahrungsmittelindustrie, der Pharmazie und Kosmetik eingesetzt. Normalerweise sind Monoglyceride W/O-Emulgatoren, doch werden sie durch kleine Zusätze (Seifen, Polyethylenoxid-Verbindungen, sulfatierte Alkohole) "selbstemulgierend" und dann gute O/W-Emulgatoren. In Abhängigkeit vom Zusatz können sie säurestabile und elektrolytstabile Emulsionen liefern. Die Monoglyceride höherer Fettsäuren (z.B. Glycerinmonostearat) dienen als Gleitmittel bei der Kunststoffverarbeitung. Durch Molekulardestillation gewonnene Monoglyceride mit einem Monoglyceridgehalt von über 90 % werden hauptsächlich in der Nahrungsmittelindustrie (Teig-, Süß- und Backhilfswaren, Margarine, Speiseeise) verwendet.

Durch Zusatz von Monoglycerid (bis zu 5 % Palmitin/Stearinsäuremonoglycerid 90%ig oder 10 % Palmitin-Stearinsäure-mono/diglycerid) erreicht man selbstemulgierende Eigenschaften der zu Backzwecken bestimmten Backfette (superglycerolated shortenings).

Der Ausdruck "shortening" bedeutet wörtlich verkürzend und geht auf die Backeigenschaften zurück. Aufgrund der besonderen Struktur vermögen die Monoglyceride, die plastifizierende Wirkung von Stärke und Gluten bei der Teigbereitung zu verändern, indem sie sich in feiner Verteilung in die homogenen Plastikate einschieben, sie unterbrechen und damit den Teig geschmeidiger machen, d.h. verkürzen. Gleichzeitig wird die Einarbeitung von Luft erleichtert, so daß zusammengenommen Backwaren mit vergrößertem Volumen und besserer "Kürze" anfallen.

Die wesentlichen Bestandteile der Margarine sind Speisefette und -öle, Trinkwasser, Emulgatoren. Als Emulgatoren dienen Lecithin, Eigelb und/oder Mono- und Diglyceride von Speisefettsäuren. Außerdem kann die Margarine noch Geruchsund Geschmackstoffe (Aromastoffe), gesäuerte Milch, Magermilch, Speisesalz, Starkesirup, Zitronensaure und/oder andere Genußsäuren, Vitamine sowie zugelassene Konservierungsmittel und zugelassene Farbstoffe (im allgemeinen Carotin oder carotinhaltige Öle) enthalten. Emulgatoren sind unerläßliche Hilfsstoffe bei der Margarineherstellung, da sie die Bildung von Wasser in Öl-Emulsionen ermöglichen. Am gebräuchlichsten sind Monoglyceride und pflanzliches Lecithin, die sich in ihrer emulgierenden Wirkung unterstützen. Zur praktischen Anwendung kommen Produkte mit etwa 40 % bzw. etwa 90 % Monoglyceriden von $C_{16}/C_{18}$-Säuren (Palmitinsäure, Stearinsäure, auch im Gemisch mit Ölsäure), die daneben 60 % bzw. 10 % Diglyceride enthalten. Zusätze von bis zu 0,5 % bzw. 0,25 %, entsprechend etwa 0,2 % Monoglycerid, bezogen auf die Fettphase, sind üblich. Zur Herstellung von Halbfettmargarine benötigt man gewöhnlich höhere Emulgatoranteile.

Die Mono- und Diglyceride sind durch Verestern von Glycerin mit Fettsäuren erhältlich. Ein anderer Weg ist die Umesterung von Triglyceriden mit Glycerin. Weiterhin hat die enzymatische Zerlegung von Triglyceriden in neuerer Zeit Eingang in die Technik gefunden. Alle Methoden führen zu einem Gemisch von Mono-, Di- und Triglyceriden. Bei der Veresterung stellt sich ein Gleichgewichtsgemisch von etwa 60 % Mono-, 35 % Di- und 5 % Tri-glyceriden ein. Das Gemisch wird durch Molekulardestillation getrennt. Bei den hohen Temperaturen im Filmverdampfer erfolgt in geringem Maße Disproportionierung, so daß Mono- oder Diglyceride kleine Mengen der anderen beiden Ester und geringfügige Mengen an freien Fettsäuren enthalten.

Wegen der Disproportionierung bei den Temperaturen im Filmverdampfer sind Monoglyceride mit Gehalten von mehr als 95 % wirtschaftlich mit der Molekulardestillation nicht erhältlich. Es besteht aber ein großes Interesse an Monoglyceriden, die eine Reinheit von 99 % und darüber besitzen. Das gelingt überraschenderweise nach dem Verfahren dieser Erfindung mit Hilfe der Extraktion mit einem überkritischen Extraktionsmittel, das aus einem leichtflüchtigen Kohlenwasserstoff mit 2 bis 6 Kohlenstoffatomen und z.B. Kohlendioxid und/oder $N_2O$ zusammengesetzt ist. Es ist bekannt, daß Monoglyceride aus einem Gemisch von Mono-, Di- und Triglyceriden mit Hilfe von dichtem Kohlendioxid abgetrennt werden können. Dazu sind jedoch Drücke von über 350 atm bei Temperaturen von 40°C erforderlich. Außerdem ist die Beladung des dichten Kohlendioxids selbst bei Drücken von 350 atm noch so gering (weniger als 1 %), daß eine wirtschaftliche Gewinnung hochprozentiger Monoglyceride nicht möglich ist.

Auch wurde die Verwendung z.B. von Aceton als Schleppmittel vorgeschlagen (DE-OS 23 40 566.5.) Dabei gelangen die Monoglyceride als die leichter löslichen Komponenten in das Kopfprodukt bei der Gegenstromextraktion. Die Trennfaktoren sind dabei jedoch relativ gering, so daß die Gewinnung reiner Monoglyceride, zumal die erreichten Beladungen mit 1 bis 1,5 % gering sind, wirtschaftlich nicht interessant ist. Außerdem ist die Entfernung des Schleppmittels Aceton aus dem Produkt aufwendig.

Gegenstand der Erfindung ist nun ein Verfahren zur Gewinnung von Monoglyceriden und Diglyceriden aus einem Gemisch, das Mono-, Di- und Triglyceride enthält, durch Extraktion im Gegenstrom mit einem überkritischen Extraktionsmittel, das dadurch gekennzeichnet ist, daß das überkritische Extraktionsmittel als Kosolvens einen Kohlenwasserstoff in einer Menge von 30 bis 90 Gew.%, vorzugsweise von 40 bis 80 Gew.%, enthält und das Extraktionsmittel während des Kreislaufes im überkritischen Bereich bleibt.

Nach dem Verfahren dieser Erfindung erscheinen im Gegensatz zu dem in der DE-OS 23 40 566.5 beschriebenen Verfahren die Monoglyceride nicht als Kopfprodukt, sondern als Sumpfprodukt in der Gegenstromkolonne. Als Extrakt enthält das Extraktionsmittel am Kopf der Kolonne die Di- und Triglyceride, die nachfolgend durch fraktionierte Ausfällung aus dem Extraktionsmittel getrennt werden können. Zur fraktionierten Ausfällung können zwei Regenerierkolonnen verwendet werden. Wenn auf fraktionierte Abscheidung der Di- und Triglyceride verzichtet wird, genügt eine Regenerierkolonne.

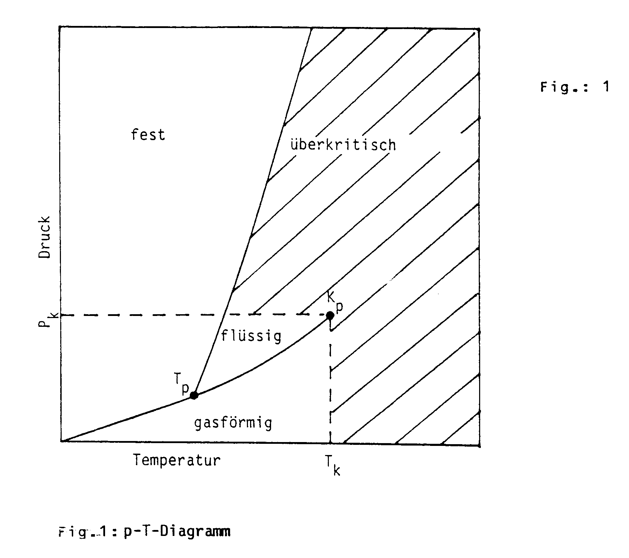

Nach dem Verfahren dieser Erfindung sind durch Verwendung z.B. von Kohlendioxid und/oder $N_2O$ als überkritisches Extraktionsmedium und der Verwendung von niedermolekularen Kohlenwasserstoffen, wie z.B. Ethan, Propan, Butan als Kosolvens, hohe Trennfaktoren verbunden mit hoher Beladung des Extraktionsmittels möglich. Dabei sollen Substanzen als in überkritischem Zustand befindlich bezeichnet werden, deren Temperatur bei entsprechenden Drücken höher als die kritische Temperatur bzw. deren Druck bei der entsprechenden Temperatur höher als der kritische Druck ist (schraffiertes Feld, gemäß Fig.1). Während bei Zusätzen von 10 Gew.% des Kosolvens nur unbedeutende Effekte beobachtet werden, durchlaufen überraschenderweise im Konzentrationsbereich von 30 bis 90 Gew.% des Kosolvens die Trennfaktoren einen Maximalwert. In diesem Bereich ist auch die erzielbare Konzentration im Extraktionsmittel an Glyceriden mit 2 bis 12 Gew.% so hoch, daß wirtschaftliches Arbeiten moglich ist. Eine Beladung des Extraktionsmittels von 6 Gew.% wird bei 40°C schon bei den geringen Drücken von 120 atm erhalten. Bei reinen Kohlenwasserstoffen sind die Trennfaktoren wieder sehr gering, d.h. nahezu 1.

In einer Gegenstromkolonne, im folgenden auch als Trennkolonne bezeichnet, in der das Extraktionsmittel z.B. aus $CO_2$ mit dem Kosolvens, z.B. Propan, von unten nach oben stömt und das zu trennende Gemisch etwa in der Mitte oder am Kopf der Kolonne zugegeben wird, fließt im Gegenstrom die flüssige Phase nach unten. Letztere verarmt auf dem Weg nach unten an Di- und Triglyceriden, bis schließlich ein Sumpfprodukt mit einem Monoglyceridgehalt von über 99 % anfällt. Das am Kopf dieser Kolonne austretende Extraktionsmittel enthält neben einem geringfügigen Rest an Monoglyceriden die im Zulauf vorhandenen Anteile an Di- und Triglyceriden. Durch schrittweise Druckverminderung in zweinachgeschalteten Abscheidern können im ersten Abscheider bzw. Regenerierkolonne bevorzugt die Diglyceride und im zweiten Abscheider bzw. Regenerierkolonne bevorzugt die Triglyceride aus dem im Kreis geführten Extraktionsmittel abgetrennt werden. Ein Teil der im ersten Abscheider ausgefällten Glyceride wird als Rücklauf auf die Trennkolonne gegeben und der. Rest als Produkt entnommen. Die gegebenenfalls im zweiten Abscheider ausgefällten Glyceride werden teilweise als Rücklauf auf den Kopf des ersten Abscheiders zurückgeführt und der Rest als weiteres Produkt, das praktisch nur Triglyceride enthält, entnommen. Das aus dem ersten Abscheider abgezogene Produkt enthält die Diglyceride. Auf diese Weise gelingt nach dem Verfahren dieser Erfindung die Auftrennung des Gemisches aus Mono-, Di- und Triglyceriden in drei Fraktionen, die jeweils die Monoglyceride, die Diglyceride und die Triglyceride in hoher Reinheit enthalten, in einem Arbeitsgang.

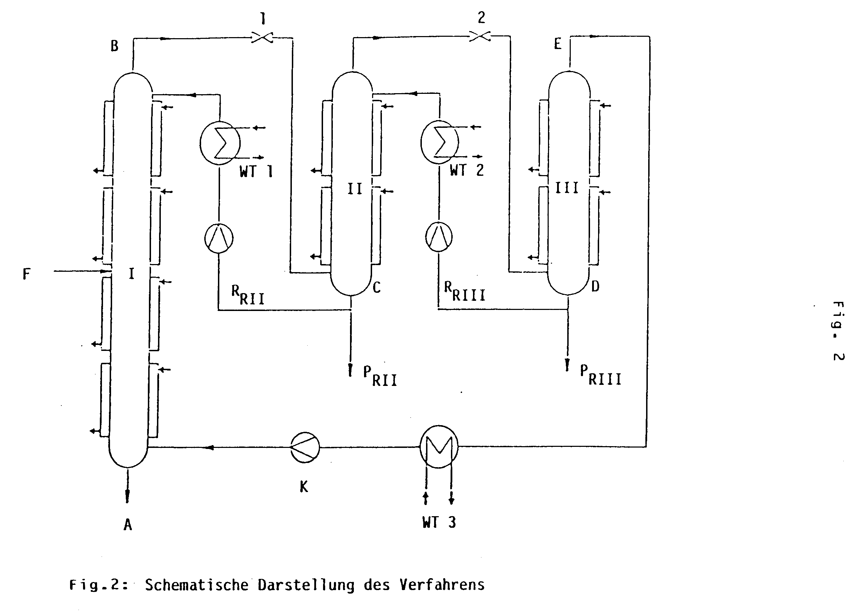

Anhand der schematischen Darstellung in Fig. 2 sei eine Ausführungsform des erfindungsgemäßen Verfahrens näher erläutert:

Die Apparatur besteht aus drei Kolonnen, von denen die eine, die Trennkolonne I zur Abtrennung der Monoglyceride aus einem Glyceridgemisch dient. Die Monoglyceride werden als Sumpfprodukt erhalten (Punkt A). Die Regenerierkolonnen II und III dienen zur Abtrennung der extrahierten Komponeten aus dem Kreisgas.

Das Glyceridgemisch wird im mittleren Teil der Trennkolonne (Punkt F) zugeführt. Das zirkulierende Extraktionsmittel beläd sich in der Trennkolonne bevorzugt mit Di- und Triglyceriden. Gleichzeitig löst sich das Extraktionsmittel in der sich mit Monoglyceriden anreichernden und nach unten strömenden Flüssigphase auf.

Das mit den leichter löslichen Komponenten Di- und Triglycerid beladene Extraktionsmittel verläßt den Verstärkerteil der Trennkolonne I am Kopf (bei Punkt B). Das beladene Extraktionsmittel wird über das Druckreduzierventil 1 in die Regenerierkolonne II entspannt. Druck und Temperatur werden in Regenerierkolonne II so gewählt, daß bevorzugt Diglyceride und der geringe Rest an Monoglyceriden abgeschieden werden. Die kondensierte Phase wird aus dem Sumpf (C) der Regenerierkolonne II abgezogen und in Produkt $P_{RII}$ und Rücklauf $R_{RII}$ geteilt. Der Rücklauf wird nach Durchlaufen des Wärmetauschers WT1 am Kopf der Trennkolonne I aufgegeben. Der mit Triglyceriden beladene Extraktionsmittelstrom aus Regenerierkolonne II wird über das Druckreduzierventil 2 in der Regenerierkolonne III entspannt. In der Regenerierkolonne III werden Druck und Temperatur so gewählt, daß das die Kolonne verlassende Extraktionsmittel frei von schwerflüchtigen Komponenten ist. Die kondensierte Phase wird aus dem Sumpf (D) der Regenerierkolonne III abgezogen und in Produkt $P_{RIII}$ und Rücklauf $R_{RIII}$ geteilt. Der Rücklauf aus Regenerierkolonne III wird nach Durchlaufen des

3

Wärmetauschers WT2 am Kopf der Regenerierkolonne II aufgegeben. Die Rücklaufschaltung ermöglicht eine Fraktionierung der Di- und Triglyceride. Aus Regenerierkolonne II wird ein diglyceridreiches Sumpfprodukt, aus Regenerierkolonne III ein triglyceridreiches Sumpfprodukt erhalten.

Die anfallenden Sumpfprodukte werden kontinuierlich abgezogen und in Behälter entspannt. Die sich dabei entlösenden Mengen an gasförmigen Lösungsmitteln werden über einen Kompressor in den Kreislauf zurückgegeben. Das regenerierte Kreisgas verläßt die Regenerierkolonne III bei Punkt E und wird mit Hilfe eines Kompressors K nach Durchlaufen des Wärmeaustauschers WT3 der Trennkolonne I wieder zugeführt.

Bei fraktionierter Abscheidung der im Extraktionsmittel gelösten Komponenten wird bei isothermer Fahrweise der Druck in der ersten Abscheidekolonne um etwa 10 bis 60 bar, vorzugsweise 20 bis 50 bar gegenüber dem Druck in der Trennkolonne vermindert. Die fraktionierte Abscheidung in der ersten Abscheidekolonne kann aber auch isobar erfolgen. In diesem Fall wird die Temperatur in der Abscheidekolonne um 10 bis 80°C, vorzugsweise um 20 bis 50°C, gegenüber der Temperatur der Trennkolonne erhöht. Eine Kombination von Verminderung des Druckes und Erhöhung der Temperatur ist ebenso möglich.

Die Abscheidebedingungen in der zweiten Abscheidekolonne entsprechen den Bedingungen bei Regenerierung des Extraktionsmittels, wenn auf Fraktionierung verzichtet wird. Die Regenerierung des Extraktionsmittels erfolgt entweder durch Entspannung allein oder durch Entspannung bei gleichzeitiger Temperaturerhöhung. Der Druck bei Regenerierung beträgt 30 bis 80 bar im Temperaturbereich von 40 bis 120°C.

Beispiel 1

Ein Glyceridgemisch aus 60,9 Gew.-% Monoglyceriden, 35,3 Gew.-% Diglyceriden, 3,4 Gew.-% Triglyceriden der Ölsäure und 0,4 Gew.-% freien Säuren wurde bei einer Temperatur von 40°C und einem Druck von 120 bar einer Gegenstromkolonne in der Mitte zugepumpt und einer Extraktion unterzogen. Dabei diente als Extraktionsmittel ein Gemisch aus 42 Gew.-% Kohlendioxid und 58 Gew.-% Propan, welches unter den genannten Arbeitsbedingungen überkritisch, d.h. einphasig ist. Die Beladung des Extraktionsmittels mit Glyceriden betrug 3,1 Gew.-%. Die Kolonne war 4 m hoch und war mit einer Sulzer Drahtnetzpackung CY ausgerüstet. Der die Trennkolonne am Kopf verlassende Extraktstrom wurde in die Regenerierkolonne überführt. Die Betriebsbedingungen der Regenerierkolonne waren 60,5 bar und 104°C. Durch gleichzeitige Erwärmung auf 104°C und Druckverminderung auf 60,5 bar nahm die Beladung des Extraktionsmittels auf 0,08 Gew.-% ab. Das so regenerierte Extraktionsmittel wurde auf 40°C gekühlt und mit Hilfe eines Umlaufkompressors in die Trennkolonne am Boden zurückgeführt. Ein Teil des in der Regenerierkolonne ausgefallenen Produktes wurde als Rücklauf der Trennkolonne am Kopf zugegeben. Das erhaltene Sumpfprodukt enthielt 98,4 Gew.-% Monoglyceride, 1,3 Gew.-% Diglyceride und 0,3 Gew.-% freie Fettsäuren. Die Konzentration an Triglyceriden lag unterhalb der Nachweisgrenze von 0,005 Gew.-%. Das in der Regenerierkolonne angefallene Kopfprodukt enthielt 31,4 Gew.-% Monoglyceride, 59,8 Gew.-% Diglyceride, 8,9 Gew.-% Triglyceride und 0,4 Gew.-% freie Fettsäuren. Es wurde nur eine Regenerierkolonne verwendet, weil auf fraktionierte Abscheidung der Di- und Triglyceride verzichtet wurde.

Beispiel 2

Das gleiche Ausgangsprodukt wie in Beispiel 1 wurde bei 20°C und 12o bar mit dem gleichen Extraktionsmittel wie im Beispiel 1 im Gegenstrom extrahiert. Die Kolonnenhöhe betrug jedoch nur 2 m. Die Regenerierung des Extraktionsmittels erfolgte bei 98°C und 57 bar. Die Beladung des Extraktionsmittels in der Trennkolonne betrug 3 Gew.-%. Bei dem hohen Lösemittelverhältnis von 30 wurden folgende Produkte erhalten: Sumpfprodukt mit 99,5 Gew.-% Monoglyceriden, 0,3 Gew.-% Diglyceriden und 0,2 Gew.-% freien Fettsäuren; Kopfprodukt mit 39,1 Gew.-% Monoglyceriden, 52,1 Gew.-% Diglyceriden, 8,4 Gew.-% Triglyceriden und 0,4 Gew.-% freien Fettsäuren.

Beispiel 3

Ein Glyceridgemisch aus 53 Gew.-% Monoglyceriden, 31 Gew.-% Diglyceriden und 13 Gew.-% Triglyceriden der Stearinsäure und 3 Gew.-% freien Fettsäuren wurde bei 140 bar und 50°C einer Gegenstromkolonne in der Mitte zugefördert. Ein Teil des in der Regenerierkolonne abgeschiedenen Produktes wurde als Rücklauf der Trennkolonne am Kopf zugegeben. Als Extraktionsmittel wurde ein Gemisch aus 70 Gew.-% Propan und 30 Gew.-% Kohlendioxid verwendet. Das unter Normalbedingungen als Feststoff vorliegende Stearinsäureglyceridgemisch ist unter den genannten Arbeitsbedingungen flüssig, das Extraktionsmittelgemisch überkritisch, d.h. einphasig. Die Höhe der Kolonne betrug 8 m. Als Einbauten wurden Sulzerpackungen Typ CY verwendet.

Die Regenerierung erfolgte bei 105°C und 55 bar. Die Beladung des Extraktionsmittels mit Glyceriden in der Trennkolonne betrug 4 Gew.-%. Bei einem Lösemittelverhältnis von 30 wurden folgende Produkte erhalten: Sumpfprodukt mit 99,0 Gew.-% Monoglyceriden, 0,4 Gew.-% Diglyceriden und 0,6 Gew.-% freien Fettsäuren; Kopfprodukt mit etwa 5 Gew.-% Monoglyceriden, 70 Gew.-% Diglyceriden, 20 Gew.-% Triglyceriden und 5 Gew.-% freien Fettsäuren.

Beispiel 4

Ein Glyceridgemisch aus 57,5 Gew.-% Monoglyceriden, 36 Gew.-% Diglyceriden, 6,15 Gew.-% Triglyceriden und 0,35 Gew.-% freien Fettsäuren, wobei die Glycerinester hauptsächlich durch Fettsäuren der Kettenlängen $C_{18}$ (Gehalt an $C_{14}$-Sauren 4 %, an $C_{16}$-Säuren 8 %), gebildet waren, wurde bei 120 bar und 40°C einer Gegenstromkolonne in der Mitte zugeführt. Als Extraktionsmittel diente ein Gemisch aus 57 Gew.-% Propan und 43 Gew.-% Kohlendioxid. Die Kolonnenhöhe betrug 16 m; als Einbauten wurden Sulzerpackungen des Typs CY verwendet. Das Extraktionsmittel mit einer Kopfbeladung an schwerflüchtigen Komponenten von ca. 10 Gew.-% wurde in die erste Regenerierkolonne bei 40°C auf 80 bar teilentspannt. Hierbei entlösen sich bevorzugt die Diglyceride und der geringe Rest an Monoglyceriden aus dem Extraktionsmittel, während die Triglyceride in Lösung bleiben. Das hauptsächlich mit Triglyceriden beladene Extraktionsmittel wurde in der zweiten Regenerierkolonne einer weiteren Entspannung auf 50 bar unterzogen, die Temperatur wurde auf 120°C angehoben. Bei diesen Bedingungen verliert das Extraktionsmittel nahezu vollständig seine Lösefähigkeit für schwerflüchtige Stoffe. Als Sumpfprodukt fiel ein triglyceridreiches Gemisch an. Das regenerierte Extraktionsmittel wurde bei der Trennkolonne am Sumpf wieder zugeführt. Zur Steigerung der Diglyceridkonzentration im Sumpf der ersten Regenerierkolonne und Minimierung der Monoglyceridverluste wurde ein Teil des Kondensates aus der ersten Abscheidekolonne am Kopf der Trennkolonne als Rücklauf aufgegeben. In analoger Weise wurde ein Teil des Kondensates aus der zweiten Regenerierkolonne als Rücklauf am Kopf der ersten Abscheidekolonne aufgegeben, um die Diglyceridkonzentration im Sumpf der ersten Regenerierkolonne zu erhöhen. Bei einem Lösemittelverhältnis von ungefähr 30 wurden folgende Produkte erhalten:

Trennkolonne:

Sumpfprodukt mit 99,2 Gew.-% Monoglycerid, 0,4 Gew.-% Diglycerid und 0,4 Gew.-% freien Fettsäuren

Erste Regenerierkolonne:

Sumpfprodukt mit 95 Gew.-% Diglycerid, 1 Gew.-% Monoglycerid, 3,7 Gew.-% Triglycerid und 0,3 Gew.-% freien Fettsäuren

Zweite Regenerierkolonne:

Sumpfprodukt mit ca. 90 Gew.-% Triglycerid, 9 Gew.-% Diglycerid, 0,8 Gew.-% Monoglycerid und 0,2 Gew.-% freien Fettsäuren.

**Patentansprüche**

1. Verfahren zur Gewinnung von Monoglyceriden und Diglyceriden aus einem Gemisch, das Mono-, Di- und Triglyceride enthält, durch Extraktion im Gegenstrom mit einem überkritischen Extraktionsmittel, dadurch gekennzeichnet, daß das überkritische Extraktionsmittel als Kosolvens einen Kohlenwasserstoff in einer Menge von 30 bis 90 Gew.-%, vorzugsweise von 40 bis 80 Gew.-%, enthält und das Extraktionsmittel während des Kreislaufes im überkritischen Bereich bleibt.

2. Verfahren nach Anspruch 1, dadurch gekennzeichnet, daß als überkritische Extraktionsmittel Kohlendioxid, $N_2O$, Schwefelhexafluorid, Trifluormethan, Tetrafluormethan oder deren Gemisch verwendet werden.

3. Verfahren nach Anspruch 1 und 2, dadurch gekennzeichnet, daß als Kosolvens Kohlenwasserstoffe mit 2 bis 6 C-Atomen oder deren Gemische verwendet werden.

4. Verfahren nach Anspruch 1 bis 3, dadurch gekennzeichnet, daß als Extraktionsmittel $CO_2$ und als Kosolvens Propan verwendet werden.

5. Verfahren nach Anspruch 1 bis 3, dadurch gekennzeichnet, daß als Extraktionsmittel $CO_2$ und als Kosolvens Butan verwendet werden.

6. Verfahren nach Anspruch 1 bis 5, dadurch gekennzeichnet, daß die Extraktion im Temperaturbereich

EP 0 352 667 B1

von 10 bis 150°C, vorzugsweise von 20 bis 80°C, ausgeführt wird.

7. Verfahren nach Anspruch 1 bis 6, dadurch gekennzeichnet, daß die Extraktion bei Drücken von 60 bis 200 atm, vorzugsweise von 80 bis 150 atm, durchgeführt wird.

8. Verfahren nach Anspruch 1 bis 7, dadurch gekennzeichnet, daß nach der Abscheidung der Monoglyceride als Sumpfprodukt in der Trennkolonne das aus Di- und Triglyceriden bestehende Extrakt durch fraktionierte Druckverminderung und Temperaturänderung in zwei aufeinanderfolgenden Abscheidern in Diglyceride und Triglyceride getrennt wird.

9. Verfahren nach Anspruch 1 bis 8, dadurch gekennzeichnet, daß zur Verbesserung der Trennung ein Teil des im ersten Abscheider anfallenden Produktes als Rücklauf auf den Kopf der Trennkolonne und ein Teil des im zweiten Abscheider anfallenden Produktes als Rücklauf auf den Kopf des ersten Abscheiders gegeben wird.

## Claims

1. Process for the recovery of monoglycerides and diglycerides from a mixture containing mono-, di- and triglycerides by extraction in countercurrent with a supercritical extractant, characterized in that the supercritical extractant contains as cosolvent a hydrocarbon in an amount of 30 to 90 weight%, preferably of 40 to 80 weight%, and that the extractant remains in the supercritical state during the cyclic process.

2. Process according to claim 1, characterized in that as supercritical extractant carbon dioxide, $N_2O$, sulfurhexafluoride, trifluoromethane, tetrafluoromethane or their mixture is used.

3. Process according to claims 1 and 2, characterized in that as cosolvent hydrocarbons having 2 to 6 carbon atoms or their mixtures are used.

4. Process according to claims 1 to 3, characterized in that as extractant $CO_2$ and as cosolvent propane are used.

5. Process according to claims 1 to 3, characterized in that as extractant $CO_2$ and as cosolvent butane are used.

6. Process according to claims 1 to 5, characterized in that the extraction is carried out in a temperature range of 10 to 150°C, preferably of 20 to 80°C.

7. Process according to claims 1 to 6, characterized in that the extraction is carried out at pressures of 60 to 200 atm, preferably of 80 to 150 atm.

8. Process according to claims 1 to 7, characterized in that after the separation of the monoglycerides as bottom product in the separation column the extract consisting of di- and triglycerides is separated into diglycerides and triglycerides by fractionated pressure decrease and change of temperature in two consecutive separators.

9. Process according to claims 1 to 8, characterized in that for an improved separation a portion of the product obtained in the first separator is fed as recycled material to the head of the separation column and a portion of the product obtained in the second separator is fed as recycled material to the head of the first separator.

## Revendications

1. Procédé d'obtention de monoglycérides et de diglycérides à partir d'un mélange contenant des mono-, des di- et des triglycérides, par extraction à contre-courant avec un agent d'extraction supercritique, caractérisé en ce que l'agent d'extraction supercritique contient, en tant que co-solvant, un hydrocarbure en une quantité de 30 à 90 et de préférence de 40 à 80 % en poids, l'agent d'extraction restant dans le domaine supercritique pendant le cycle.

2. Procédé selon la revendication 1, caractérisé en ce qu'on utilise comme agent d'extraction supercritique l'anhydride carbonique, le $N_2O$, l'hexafluorure de soufre, le trifluorométhane, le tétrafluorométhane ou leurs mélanges.

3. Procédé selon la revendication 1 et 2, caractérisé en ce qu'on utilise comme co-solvant des hydrocarbures ayant 2 à 6 atomes de carbone, ou leurs mélanges.

4. Procédé selon les revendications 1 à 3, caractérisé en ce qu'on utilise comme agent d'extraction du $CO_2$ et comme co-solvant du propane.

5. Procédé selon les revendications 1 à 3, caractérisé en ce qu'on utilise comme agent d'extraction du $CO_2$ et comme co-solvant du butane.

6. Procédé selon les revendications 1 à 5, caractérisé en ce que l'extraction est mise en oeuvre sur l'intervalle de températures de 10 à 150°C, de préférence de 20 à 80°C.

7. Procédé selon les revendications 1 à 6, caractérisé en ce que l'extraction est mise en oeuvre sous des pressions de 60 à 200 et de préférence de 80 à 150 atm.

8. Procédé selon les revendications 1 à 7, caractérisé en ce que, après séparation des monoglycérides, on sépare en tant que produit de fond de la colonne de séparation, l'extrait, constitué de di- et de triglycérides, en diglycérides et en triglycérides dans deux séparateurs successifs, par diminution fractionnée de la pression et modification fractionnée de la température.

9. Procédé selon les revendications 1 à 8, caractérisé en ce que, pour améliorer la séparation, une partie du produit obtenu dans le premier séparateur est, en tant que reflux, envoyée en tête de la colonne de séparation, et qu'une partie du produit obtenu dans le deuxième séparateur est, en tant que reflux, envoyé en tête du premier séparateur.

Fig.: 1

Fig.-1: p-T-Diagramm

Fig. 2

Fig.2: Schematische Darstellung des Verfahrens